(19) Europäisches Patentamt
European Patent Office
Office européen des brevets

(11) **EP 3 666 748 B1**

(12) **EUROPÄISCHE PATENTSCHRIFT**

(45) Veröffentlichungstag und Bekanntmachung des Hinweises auf die Patenterteilung:
**31.07.2024  Patentblatt 2024/31**

(21) Anmeldenummer: **18212259.8**

(22) Anmeldetag: **13.12.2018**

(51) Internationale Patentklassifikation (IPC):
**C07C 2/10** *(2006.01)*     **C07C 11/02** *(2006.01)*

(52) Gemeinsame Patentklassifikation (CPC):
(C-Sets verfügbar)
**C07C 2/10;** C07C 2521/12; C07C 2523/755

(Forts.)

(54) **VERFAHREN ZUR OLIGOMERISIERUNG MIT TROCKENER HANDHABUNG DES KATALYSATORS VOR DEM EINFÜLLEN IN DEN REAKTOR**

METHOD FOR OLIGOMERIZATION WITH DRY HANDLING OF THE CATALYST BEFORE DISPENSING INTO THE REACTOR

PROCÉDÉ D'OLIGOMÉRISATION À TRAITEMENT À SEC DU CATALYSEUR AVANT LE REMPLISSAGE DANS LE RÉACTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.06.2020  Patentblatt 2020/25**

(73) Patentinhaber: **Evonik Oxeno GmbH & Co. KG**
**45772 Marl (DE)**

(72) Erfinder:
• **PEITZ, Stephan**
  **45739 Oer-Erkenschwick (DE)**
• **STOCHNIOL, Guido**
  **45721 Haltern am See (DE)**
• **BUKOHL, Reiner**
  **45770 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 0 329 305     WO-A1-2010/057905**

(52) Gemeinsame Patentklassifikation (CPC): (Forts.)

C-Sets
**C07C 2/10, C07C 11/02**

# EP 3 666 748 B1

**Beschreibung**

Die vorliegende Erfindung betrifft ein Verfahren zur Oligomerisierung von kurzkettigen Olefinen in Anwesenheit eines Katalysators, wobei der Katalysator vor Einsatz im Verfahren in einer trockenen Atmosphäre gehalten wird.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Die bei der Oligomerisierung von Olefinen eingesetzten Katalysatoren weisen hohe Aktivitäten auf und sind zum Teil genau an das jeweilige Oligomerisierungsverfahren angepasst.

In der WO 2010/057805 A1 wird beispielsweise ein Verfahren zur Oligomerisierung von C2- bis C8-Olefinen, bei dem der Katalysator nach der Herstellung durch Überleiten eines Inertgasstroms behandelt, bis der abströmende Inertgasstrom einen Wassergehalt von weniger als 1000 ppm aufweist. Der Katalysator wird durch das dort beschriebene Verfahren getrocknet.

Das erste Einfüllen des Oligomerisierungskatalysators in den Reaktor oder der Austausch des vorliegenden Katalysators wird üblicherweise bei normalen Umgebungsbedingungen von Druck (ca. 1013 mbar), Temperatur (Außentemperatur, ca. 0 bis 40 °C) und Luftfeuchtigkeit (durchschnittlich > 70%) durchgeführt. Auch bei der Lagerung und beim Transport des Katalysators vom Ort der Herstellung zum Reaktor als Einsatzort herrschen üblicherweise normale Umgebungsbedingungen. Nach dem Einfüllen des Oligomerisierungskatalysators in den Reaktor wird der Reaktor angefahren, d.h. die Belastung des Katalysators mit dem zu oligomerisierenden Einsatzgemisch langsam erhöht.

Das Problem dabei ist, dass selbst bei voller Belastung des Katalysators es mehrere Tage bis Wochen dauern kann, bis mit dem neu eingebauten oder ausgetauschten Oligomerisierungskatalysator ein gewünschter Zielumsatz der Oligomerisierung erreicht werden kann.

Die Aufgabe der vorliegenden Erfindung war die Bereitstellung eines Verfahrens, mit dem die Anfahrzeit des Oligomerisierungskatalysators verringert werden kann. Die zugrundeliegende Aufgabe der vorliegenden Erfindung konnte mit dem Verfahren zur Oligomerisierung gemäß Anspruch 1 gelöst werden. Bevorzugte Ausgestaltungen sind in den Unteransprüchen angegeben.

Das erfindungsgemäße Verfahren ist ein Verfahren zur Oligomerisierung von C3- bis C6-Olefinen, wobei die Oligomerisierung in mindestens einer Reaktionsstufe, die jeweils aus mindestens einem Reaktor und mindestens einer Destillationskolonne besteht, unter Verwendung eines Oligomerisierungskatalysators erfolgt, dadurch gekennzeichnet, dass der Oligomerisierungskatalysator vor dem Einfüllen in die Reaktionsstufe bei Lagerung, Transport und/oder beim Einfüllen in die Reaktionsstufe in einer trockenen Atmosphäre bei weniger als 60 % relativer Luftfeuchtigkeit gehalten wird, wobei der Oligomerisierungskatalysator vor dem Einfüllen in die Reaktionsstufe bei Lagerung, Transport und/oder beim Einfüllen in die Reaktionsstufe in einer trockenen Atmosphäre gehalten wird, deren Luftfeuchtigkeit um mindestens 3% geringer ist als die Luftfeuchtigkeit der Umgebung und wobei der Oligomerisierungskatalysator der ersten Reaktionsstufe und der Oligomerisierungskatalysator der zweiten Reaktionsstufe jeweils unabhängig voneinander eine Nickelverbindung auf einem Alumosilicat-Trägermaterial umfasst oder daraus besteht.

Der Begriff "Reaktionsstufe" umfasst im Sinne der vorliegenden Erfindung eine Einheit aus einem oder mehreren Reaktoren und einer oder mehreren Destillationskolonnen. In den Destillationskolonnen werden insbesondere das verbliebene Einsatzgemisch, welches beispielsweise Alkane und nicht umgesetzte Olefine umfasst, von den erzeugten Produktoligomeren getrennt. Übliche verfahrenstechnische Nebenaggregate, die in den Reaktionsstufen eingebaut sein können, wie Vorwärmer für den Feed, Wärmetauscher oder ähnliches, werden hier nicht separat aufgeführt, sondern sind dem Fachmann geläufig.

Mit dem erfindungsgemäßen Verfahren hat sich überraschenderweise gezeigt, dass, wenn man bei der Handhabung, also bei Lagerung oder Transport des Oligomerisierungskatalysators vor dem Einfüllen in die Reaktionsstufe und/oder beim Einfüllen in die Reaktionsstufe auf eine trockene Atmosphäre, d. h. eine relative Luftfeuchtigkeit von weniger als 60%, bevorzugt von weniger als 50%, achtet, sich eine verringerte Anfahrzeit des Katalysators nach dem Einfüllen in den Reaktor erreichen lässt.

Der Oligomerisierungskatalysator wird vor dem Einfüllen in die Reaktionsstufe bei Lagerung, Transport und/oder beim Einfüllen in die Reaktionsstufe in einer trockenen Atmosphäre gehalten, deren Luftfeuchtigkeit um mindestens 3%, vorzugsweise um mindestens 5%, besonders bevorzugt um mindestens 10% geringer ist als die Luftfeuchtigkeit der Umgebung. Mit Umgebung ist im Sinne der vorliegenden Erfindung die Luftfeuchtigkeit in der geographischen Region gemeint, in der der Oligomerisierungskatalysator gehandhabt wird.

Oligomerisierungskatalysatoren werden typischerweise nicht direkt im Reaktor hergestellt. Die frisch hergestellten Oligomerisierungskatalysatoren müssen deshalb zunächst gelagert werden, bevor sie zur Anlage transportiert und in eine Reaktionsstufe eingefüllt werden. Das Einfüllen in die Reaktionsstufe meint insbesondere das Einfüllen in den einen oder mehrere Reaktoren der jeweiligen Reaktionsstufe. Das erfindungsgemäße Verfahren bezieht sich auf alle Einfüllvorgänge einer Reaktionsstufe bei der Oligomerisierung, d.h. das erstmalige Befüllen einer Reaktionsstufe bzw. eines Reaktors und den Austausch eines vorhandenen, gebrauchten Oligomerisierungskatalysators durch einen frischen Oligomerisierungskatalysator. Der Begriff frischer Oligomerisierungskatalysator meint im Sinne der vorliegenden Erfindung sowohl neu hergestellte Katalysatoren als auch regenerierte Katalysatoren, die bereits in einem Reaktor zur Oligomerisierung verwendet worden sind, nach dem Ausbau aber durch die nachfolgend beschriebenen Verfahren regeneriert worden sind.

Die beanspruchte Vorgehensweise ermöglicht das Erreichen des erwünschten Umsatzes bereits nach wenigen Stunden. Im Gegensatz dazu kann ein Katalysator, der bei einer Luftfeuchtigkeit von mehr als 65% und/oder vorzugsweise der bei einer Luftfeuchtigkeit, die weniger als 3% geringer ist als die Luftfeuchtigkeit der Umgebung, gelagert, transportiert und/oder eingefüllt worden ist, mehrere Tage bis zu einigen Wochen benötigen, um den gewünschten Zielumsatz zu erreichen.

Das erfindungsgemäße Verfahren wird ganz allgemein wie folgt durchgeführt. Ausgangspunkt ist ein Verfahren zur Oligomerisierung von Olefinen in einer oder mehreren, d. h. mindestens zwei Reaktionsstufen. Der für die Oligomerisierung verwendete Katalysator wird nach der Herstellung und vor dem Einfüllen in den oder die Reaktoren bei Lagerung, Transport und/oder beim Einfüllen in den oder die Reaktoren bei einer Luftfeuchtigkeit kleiner 60% gehalten.

Nach einen Zeitraum von 3 bis 6 Jahren nimmt die Katalysatoraktivität durch Belegung und/oder Vergiftung der aktiven Zentren ab und der Oligomerisierungskatalysators muss ausgetauscht werden. Dazu wird das Einsatzgemisch, welches die zu oligomerisierenden Olefine enthält, abgestellt oder einer anderen Reaktionsstufe zugeführt. Aus dem bzw. den nun nicht mehr mit Olefinen beschickten Reaktor(en) kann der Oligomerisierungskatalysator entnommen werden. Dazu wird der oder die Reaktor(en) der umfahrenen Reaktionsstufe zunächst mit einem Inertgas gespült, um flüchtige organische Substanzen zu entfernen. Danach wird der Reaktor/die Reaktoren geöffnet und der Katalysator entnommen. Sobald der Oligomerisierungskatalysator aus dem oder den Reaktoren entfernt worden ist, wird dieser mit einem frischen Katalysator unter den beanspruchten Bedingungen befüllt. Danach wird der Reaktor oder die Reaktoren inertisiert, um Sauerstoff im Reaktor zu vermeiden, angefahren und damit in den Betrieb überführt.

Das erfindungsgemäße Verfahren umfasst mindestens eine Reaktionsstufe. In einer bevorzugten Ausführungsform umfasst das Verfahren zur Oligomerisierung maximal fünf Reaktionsstufen. Insbesondere bevorzugt ist eine Verfahrensführung mit zwei, drei, vier oder fünf Reaktionsstufen. Jede dieser Reaktionsstufen umfasst unabhängig voneinander einen oder mehreren Reaktoren und eine oder mehrere Destillationskolonnen, um die gebildeten Oligomere von dem restlichen Einsatzgemisch abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Als Olefine für das erfindungsgemäße Verfahren werden C3- bis C6-Olefine, vorzugsweise C3- bis C5-Olefine, besonders bevorzugt C4-Olefine oder darauf basierende Olefingemische, die auch Anteile an analogen Alkanen enthalten können, eingesetzt. Geeignete Olefine sind unter anderem $\alpha$-Olefine, n-Olefine und Cycloalkene, vorzugsweise n-Olefine. In einer bevorzugten Ausführungsform handelt es sich bei dem Olefin um n-Buten.

Die Olefine werden üblicherweise nicht in reiner Form als Edukte eingesetzt, sondern in technisch verfügbaren Mischungen. Der in dieser Erfindung zusätzlich verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol.

Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete Olefingemische sind insbesondere das Raffinat I und das Raffinat II und das Raffinat III.

Der eine oder die Reaktoren in der oder den jeweiligen Reaktionsstufen enthalten jeweils einen Oligomerisierungskatalysator, insbesondere einen heterogenen Oligomerisierungskatalysator. Der Oligomerisierungskatalysator liegt dabei insbesondere in Form eines Granulats, eines Extrudats oder in Tablettenform vor.

Die (heterogenen) Oligomerisierungskatalysatoren in den einzelnen Reaktoren der einen oder mehreren Reaktionsstufe(n) können jeweils unabhängig voneinander aus Übergangsmetall-haltigen Oligomerisierungskatalysatoren ausgewählt werden. Die Übergangsmetalle bzw. die entsprechend eingesetzten Übergangsmetallverbindungen sind dabei vorzugsweise auf einem Trägermaterial, einem auf Alumosilicat basierenden Trägermaterial, angeordnet. Als Übergangsmetallverbindungen für die erfindungsgemäße eingesetzten Oligomerisierungskatalysatoren werden Nickel-Verbindungen eingesetzt.

Der erfindungsgemäße Oligomerisierungskatalysator umfasst eine Nickelverbindung, vorzugsweise Nickeloxid, auf einem Alumosilicat-Trägermaterial. Das Trägermaterial kann ein amorphes, mesoporöses Alumosilicat, ein kristallines, mikroporöses Alumosilicat oder eine Alumosilicat, welches amorphe und kristalline Phasen aufweist sein. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Silica-Alumina-Supportmaterial kleine kristalline Domänen aufweist.

Erfindungsgemäß weiterhin bevorzugt weist der Oligomerisierungskatalysator eine Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_2$ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. In einer besonders bevorzugten Ausführungsform der vorliegenden Erfindung ist der Oligomerisierungskatalysator im Wesentlichen frei von Titandioxid und/oder Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumdioxid in seiner Gesamtzusammensetzung.

Der Oligomerisierungskatalysator weist vorzugsweise eine spezifische Oberfläche (berechnet nach BET) von 150 bis 700 $m^2$/g, weiterhin bevorzugt von 190 bis 600 $m^2$/g, besonders bevorzugt von 220 bis 550 $m^2$/g aufweisen. Die BET-Oberfläche wird mittels Stickstoff-Physisorption gemäß DIN-ISO 9277 (Stand: 2014-01) gemessen.

Die in den einzelnen Reaktoren in der oder den Reaktionsstufe(n) vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre (Regenerierte Katalysatoren verhalten sich anders als neu hergestellte Katalysatoren, Abrieb während des Betriebes, unterschiedlich schnelle Alterung und/oder Vergiftung, etc.) ändern.

Mit steigender Einsatzzeit des Oligomerisierungskatalysators bei der Oligomerisierung kann es zu einer Abnahme des Umsatzes und/oder der Selektivität (allgemein der Katalysatoraktivität) durch Altern und/oder Vergiftung kommen. Der erfindungsgemäße Katalysator wird dann ausgetauscht und durch einen frischen Oligomerisierungskatalysator, also einen neuen oder einen regenerierten Oligomerisierungskatalysator, ersetzt.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Nach dem Einsatz des hergestellten Oligomerisierungskatalysators in der Oligomerisierung kann der Katalysator auch regeneriert werden, um die Katalysatoraktivität (für die Oligomerisierung) wieder zu steigern. Die Regenerierung des Oligomerisierungskatalysators umfasst insbesondere die Schritte A) des Abbrennens und B) des Imprägnierens mit einer Lösung einer Übergangsmetallverbindung. Dabei sollten das Übergangsmetall in dem zu regenerierenden Katalysator und das Übergangsmetall in der Imprägnierlösung identisch sein.

Es ist möglich, dass der Oligomerisierungskatalysator nach seinem Einsatz in der Oligomerisierung Ablagerungen organischer Stoffe aufweist, die entfernt werden müssen. Die Entfernung der im Katalysator abgelagerten organischen Verbindungen geschieht vorzugsweise in Schritt A) durch einfaches Abbrennen, wodurch Kohlenstoffoxide und Wasser entstehen. Das Abbrennen in Schritt A) kann in einem Ofen, beispielweise in einem Drehrohrofen oder einem Schachtofen, kontinuierlich oder diskontinuierlich durchgeführt werden. Dazu wird der Oligomerisierungskatalysators, beispielsweise in Form eines Granulats, dem Ofen zugeführt und vorzugsweise bei einer Ofentemperatur von 400 bis 600 °C, besonders bevorzugt von 500 bis 600 °C abgebrannt. Das Abbrennen kann unter Einsatz von zusätzlich zugeleiteter Verbrennungsluft durchgeführt werden. Diese Verbrennungsluft kann im Gegenstrom zugeführt werden, optional wird zusätzlich weitere Luft über geeignete Einlässe in den Oligomerisierungskatalysator eingeblasen werden, um einen schnellen Abbrand zu gewährleisten.

Schritt B) umfasst als Schritt B1) eine Beaufschlagung/Imprägnierung des in Schritt A) abgebrannten Oligomerisierungskatalysators mit einer Lösung einer Übergangsmetallverbindung, insbesondere einer Lösung einer Nickelverbindung und als Schritt B2) eine Kalzination des beaufschlagten Oligomerisierungskatalysators.

Im Folgenden wird die Durchführung des Schritts B1) der Regeneration am Beispiel von Nickel diskutiert. Die Aussagen sind aber auch auf andere Übergangsmetall übertragbar.

Die Imprägnierung mit Nickel in Schritt B1) kann ähnlich wie die Herstellung des Oligomerisierungskatalysators erfolgen, optional jedoch mit dem Unterschied, dass eine Nickellösung mit einer geringeren Nickelkonzentration als bei der Herstellung des Oligomerisierungskatalysators verwendet werden kann. Prinzipiell kann dazu jede lösliche Nickelverbindung wie Nickelnitrat $(Ni(NO_3)_2)$, Nickelacetat $(Ni(ac)_2)$, Nickelacetylacetonat $(Ni(acac)_2)$, Nickelsulfat $(NiSO_4)$ oder Nickelcarbonat $(NiCOs)$ zur Herstellung einer wässrigen oder ammoniakalischen Nickel-Lösung verwendet werden.

Als besonders vorteilhaft hat sich die Verwendung von NiHAC-Lösungen erwiesen, die durch Auflösen von Nickelcarbonat $(NiCOs)$ in konzentrierten Ammoniaklösungen ggf. mit Zusatz von Ammoniumcarbonat erhältlich sind. Derartige Lösungen können mit Nickelgehalten von 0,5 bis 14 Gew.-%, insbesondere von 2 bis 10 Gew.-%, ganz besonders bevorzugt von 4 bis 8 Gew.-% für die Imprägnierung verwendet werden.

Zum Nickelauftrag wird der in Schritt A) abgebrannte Oligomerisierungskatalysator beispielsweise mit einer NiHAC-Lösung mit Nickelgehalten von 0,5 bis 14 Gew.-%, insbesondere von 2 bis 10 Gew.-%, ganz besonders von 4 bis 8 Gew.-% bis zur Sättigung der im Oligomerisierungskatalysator vorhandenen Poren imprägniert. Die Imprägnierung kann mit einem dem Fachmann geläufigen Verfahren wie beispielsweise durch Besprühen bis zum permanenten Auftreten eines Flüssigkeitsfilmes auf der Oberfläche (incipient wetness) durchgeführt werden. Beträgt die Lösungsaufnahme etwa 0,8 bis 1,2 g Lösung pro g Oligomerisierungskatalysator, kann man erreichen, dass etwa 0,5 bis 6 Gew.-% an zusätzlichem Nickel in Form eines basischen Carbonates abgeschieden werden.

Nach dem Schritt B1) kann der imprägnierte Oligomerisierungskatalysator in einer geeigneten Trocknungsapparatur, beispielsweise einem Bandtrockner mit Luftstrom oder auch einem Konustrockner, bei Temperaturen zwischen 100 und 250 °C, vorzugsweise zwischen 120 und 220 °C und Normaldruck oder auch im Vakuum getrocknet werden bevor die Kalzination in Schritt B2) erfolgt. Durch die Trocknung wird insbesondere Wasser oder überschüssigen Ammoniak aus der NiHAC-Lösung ausgetragen.

Die Kalzination des Oligomerisierungskatalysators in Schritt B2) kann in einem geeigneten Ofen, wie beispielsweise einem Schachtofen oder Drehrohrofen, kontinuierlich oder diskontinuierlich durchgeführt werden. Bei einer kontinuierlichen Kalzination wird weiterhin bevorzugt ein Gas im Gegenstrom durch den Oligomerisierungskatalysator, insbesondere in Form eines Granulats geleitet. Als Gas kann Luft, Stickstoff oder eine Mischung davon verwendet werden. Der Gasstrom kann 0,2 bis 4 $m^3$ Gas pro kg Granulat und Stunde und die Eintrittstemperatur des Gases von 400 bis 800 °C, vorzugsweise 450 bis 700 °C betragen. Zusätzlich zu dieser über das Gas eingetragenen Wärme kann Energie durch aktives Heizen der Wände des Ofens eingetragen werden.

Die Kalzinationstemperatur in dem Ofen kann in Schritt B2) 400 bis 800 °C, vorzugsweise 450 bis 700 °C, besonders bevorzugt 500 bis 600 °C betragen. Diese Temperatur kann über mehrere Stunden, vorzugsweise 5 bis 60 Stunden, besonders bevorzugt 10 bis 40 Stunden gehalten werden, bevor der Katalysator abgekühlt wird. Das Abkühlen findet vorzugsweise im Stickstoffstrom statt. Dem Stickstoff kann zusätzlich Luft hinzugefügt werden, wobei die Luftmenge vorzugsweise kontrolliert werden sollte. Die Menge an Luft, die dem Stickstoff vorzugsweise hinzugefügt wird, kann 100 bis 10000 Vol.-ppm, bevorzugt 300 bis 7000 Vol.-ppm betragen.

Die Oligomerisierung des erfindungsgemäßen Verfahren kann bei einer Temperatur im Bereich von 50 bis 200 °C, vorzugsweise 60 bis 180 °C, bevorzugt im Bereich von 60 bis 130°C, und bei einem Druck von 10 bis 70 bar, bevorzugt von 20 bis 55 bar durchgeführt werden. In einer bevorzugten Ausführungsform der vorliegenden Erfindung wird die Oligomerisierung in flüssiger Phase durchgeführt. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 $h^{-1}$) und 190 $h^{-1}$, vorzugsweise zwischen 2 $h^{-1}$ und 35 $h^{-1}$, insbesondere bevorzugt zwischen 3 $h^{-1}$ und 25 $h^{-1}$.

In einer Ausführungsform, insbesondere bei Verwendung eines Katalysators, welcher ein Nickelverbindung, vorzugsweise Nickeloxid, auf einem Alumosilicat-Trägermaterial umfasst, beträgt der Grad der Dimerisierung (auch "prozentuale Selektivität bezogen auf die Dimerisierung" genannt) nach der Oligomerisierung bezogen auf das umgesetzte Edukt mindestens 60 %, weiter bevorzugt mindestens 75 %, besonders bevorzugt mindestens 80%.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel:

$$\frac{(\text{einfach verzweigte Dimere (Gew.-\%)} + 2 \times \text{zweifach verzweigte Dimere (Gew.-\%)})}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das $C_9$-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen. Nachfolgend wird die vorliegende Erfindung anhand von Beispielen näher erläutert. Alternative Ausführungsformen der vorliegenden Erfindung sind in analoger Weise erhältlich.

Beispiel 1 (erfindungsgemäß):

Ein Nickel(oxid)-haltiger Alumosilikat-Katalysator mit 20 Gew.-% Nickel wird nach der Herstellung und vor dem Einfüllen in den Reaktor in einem luftdicht verschlossenen Gefäß drei Monate gelagert. Vor dem Einfüllen in den Reaktor wird er 24 Stunden an Raumluft bei 50 % relativer Luftfeuchtigkeit gehalten. Der Reaktor wird bei 80°C und 30 bar mit einem Massenstrom von 1 kg/h C4-Gemisch befahren, der Butengehalt beträgt 72%. Zu diesem Frischzulauf wird zusätzlich 1 kg/h Reaktoraustrag recycliert, so dass der Gesamtzulauf 2 kg/h beträgt. Nach 6 Stunden Laufzeit erreicht der Umsatz an Butenen ein konstantes Niveau von 49%. Nach Anheben der Reaktionstemperatur auf 90°C liegt der Umsatz bei 50,5%.

Beispiel 2 (nicht erfindungsgemäß):

Ein Oligomerisierungskatalysator (gleicher Katalysator wie in Beispiel 1) wird nach der Herstellung in einem luftdicht verschlossenen Gefäß drei Monate gelagert. Vor dem Einfüllen in den Reaktor wird er 24 Stunden an Raumluft bei 95 % relativer Luftfeuchtigkeit gehalten. Der Reaktor wird bei 90°C und 30 bar mit einem Massenstrom von 1 kg/h C4-Gemisch befahren, der Butengehalt beträgt 75%. Zu diesem Frischzulauf wird zusätzlich 1 kg/h Reaktoraustrag recycliert, so dass der Gesamtzulauf 2 kg/h beträgt. Nach 46 Stunden Laufzeit wird ein Umsatz von 35% erreicht. Erst nach 140 Stunden Laufzeit erreicht der Umsatz an Butenen ein konstantes Niveau von 50,5%.

Beispiel 3 (nicht erfindungsgemäß):

Ein Oligomerisierungskatalysator (gleicher Katalysator wie in Beispiel 1) wird nach der Herstellung in einem luftdicht verschlossenen Gefäß drei Monate gelagert. Vor dem Einfüllen in den Reaktor wird er 4,5 Wochen an Raumluft bei 95 % relativer Luftfeuchtigkeit gehalten. Der Reaktor wird bei 90°C und 30 bar mit einem Massenstrom von 1 kg/h C4-Gemisch befahren, der Butengehalt beträgt 75%. Zu diesem Frischzulauf wird zusätzlich 1 kg/h Reaktoraustrag recycliert, so dass der Gesamtzulauf 2 kg/h beträgt. Nach 436 Stunden Laufzeit wird ein Umsatz von 1%, nach 555 h von 26,4% erreicht. Nach 795 Stunden Laufzeit erreicht der Umsatz an Butenen ein konstantes Niveau von 39%.

Die Versuche zeigen deutlich, dass die Lagerung an Luft bei hoher Luftfeuchtigkeit zu Problemen beim Anfahren führt und ausreichende Umsätze, wie beim erfindungsgemäßen Beispiel, gar nicht oder erst nach langen Einsatzzeiten erreicht werden können.

**Patentansprüche**

1. Verfahren zur Oligomerisierung von C3- bis C6-Olefinen, wobei die Oligomerisierung in mindestens einer Reaktionsstufe, die jeweils aus mindestens einem Reaktor und mindestens einer Destillationskolonne besteht, unter Verwendung eines Oligomerisierungskatalysators erfolgt, **dadurch gekennzeichnet, dass** der Oligomerisierungskatalysator vor dem Einfüllen in die Reaktionsstufe bei Lagerung, Transport und/oder beim Einfüllen in die Reaktionsstufe in einer trockenen Atmosphäre bei weniger als 60% relativer Luftfeuchtigkeit gehalten wird, wobei der Oligomerisierungskatalysator vor dem Einfüllen in die Reaktionsstufe bei Lagerung, Transport und/oder beim Einfüllen in die Reaktionsstufe in einer trockenen Atmosphäre gehalten wird, deren Luftfeuchtigkeit um mindestens 3% geringer ist als die Luftfeuchtigkeit der Umgebung und wobei der Oligomerisierungskatalysator der ersten Reaktionsstufe und der Oligomerisierungskatalysator der zweiten Reaktionsstufe jeweils unabhängig voneinander eine Nickelverbindung auf einem Alumosilicat-Trägermaterial umfasst oder daraus besteht.

2. Verfahren zur Oligomerisierung nach Anspruch 1, wobei der frische Oligomerisierungskatalysator bei Lagerung, Transport und/oder Befüllung der Reaktionsstufe in einer trockenen Atmosphäre bei weniger als 50% relativer Luftfeuchtigkeit gehalten wird.

3. Verfahren nach Anspruch 1, wobei der Oligomerisierungskatalysator eine Zusammensetzung von 15 bis 40 Gew.-% NiO, 5 bis 30 Gew.-% $Al_2O_3$, 55 bis 80 Gew.-% SiOz und 0,01 bis 2,5 Gew.-% eines Alkalimetalloxids aufweist-

4. Verfahren zur Oligomerisierung nach einem der Ansprüche 1 bis 3, wobei das Verfahren zwei, drei, vier oder fünf Reaktionsstufen umfasst.

5. Verfahren zur Oligomerisierung nach einem der Ansprüche 1 bis 4, wobei der zum Einfüllen verwendete Oligomerisierungskatalysator ein neu hergestellter Oligomerisierungskatalysator oder ein regenerierter Oligomerisierungskatalysator ist.

6. Verfahren zur Oligomerisierung nach Anspruch 5, wobei der regenerierte Oligomerisierungskatalysator durch die Schritte A) Abbrennen eines für die Oligomerisierung benutzten Oligomerisierungskatalysators und B) Imprägnieren des in Schritt A) abgebrannten Oligomerisierungskatalysators mit einer Lösung einer Übergangsmetallverbindung und anschließender Kalzination hergestellt wird.

7. Verfahren zur Oligomerisierung nach einem der Ansprüche 1 bis 6, wobei die Oligomerisierung bei einer Temperatur im Bereich von 50 bis 200 °C und bei einem Druck von 10 bis 70 bar durchgeführt wird.

**Claims**

1. Process for the oligomerization of C3- to C6-olefins, wherein the oligomerization is effected in at least one reaction stage, which consists in each case of at least one reactor and at least one distillation column, using an oligomerization catalyst, **characterized in that** the oligomerization catalyst before being charged to the reaction stage in storage, transport and/or in charging to the reaction stage is kept in a dry atmosphere at less than 60% relative humidity, wherein the oligomerization catalyst before being charged to the reaction stage in storage, transport and/or in charging to the reaction stage is kept in a dry atmosphere having a humidity that is at least 3% lower than the humidity of the surroundings and wherein the oligomerization catalyst of the first reaction stage and the oligomerization catalyst of the second reaction stage each independently comprises or consists of a nickel compound on an aluminosilicate support material.

2. Process for the oligomerization according to Claim 1, wherein the fresh oligomerization catalyst in storage, transport and/or charging to the reaction stage is kept in a dry atmosphere at less than 50% relative humidity.

3. Process according to Claim 1, wherein the oligomerization catalyst has a composition of 15% to 40% by weight of NiO, 5% to 30% by weight of $Al_2O_3$, 55% to 80% by weight of $SiO_2$ and 0.01% to 2.5% by weight of an alkali metal oxide.

4. Process for the oligomerization according to any of Claims 1 to 3, wherein the process comprises two, three, four or five reaction stages.

5. Process for the oligomerization according to any of Claims 1 to 4, wherein the oligomerization catalyst used for charging is a newly produced oligomerization catalyst or a regenerated oligomerization catalyst.

6. Process for the oligomerization according to Claim 5, wherein the regenerated oligomerization catalyst is produced by the steps A) of burning off an oligomerization catalyst used for the oligomerization and B) of impregnating the oligomerization catalyst burnt off in step A) with a solution of a transition metal compound and subsequent calcining.

7. Process for the oligomerization according to any of Claims 1 to 6, wherein the oligomerization is carried out at a temperature in the range of 50°C to 200°C and at a pressure of 10 to 70 bar.

**Revendications**

1. Procédé d'oligomérisation d'oléfines en C3-C6, l'oligomérisation étant effectuée dans au moins un étage de réaction qui est constitué à chaque fois par au moins un réacteur et au moins une colonne de distillation, à l'aide d'un catalyseur d'oligomérisation, **caractérisé en ce que** le catalyseur d'oligomérisation est maintenu, avant l'introduction dans l'étage de réaction, lors du stockage, du transport et/ou de l'introduction dans l'étage de réaction, dans une atmosphère sèche à moins de 60% d'humidité relative de l'air, le catalyseur d'oligomérisation étant maintenu, avant l'introduction dans l'étage de réaction, lors du stockage, du transport et/ou lors de l'introduction dans l'étage de réaction, dans une atmosphère sèche dont l'humidité de l'air est inférieure d'au moins 3% à l'humidité de l'air de l'environnement et le catalyseur d'oligomérisation du premier étage de réaction et le catalyseur d'oligomérisation du deuxième étage de réaction comprenant ou étant constitué par, à chaque fois indépendamment l'un de l'autre, un composé de nickel sur un matériau support d'aluminosilicate.

2. Procédé d'oligomérisation selon la revendication 1, le catalyseur d'oligomérisation frais étant maintenu lors du stockage, du transport et/ou du remplissage de l'étage de réaction dans une atmosphère sèche à moins de 50% d'humidité relative de l'air.

3. Procédé selon la revendication 1, le catalyseur d'oligomérisation présentant une composition de 15 à 40% en poids de NiO, 5 à 30% en poids d'$Al_2O_3$, 55 à 80% en poids de $SiO_2$ et 0,01 à 2,5% en poids d'un oxyde de métal alcalin.

4. Procédé d'oligomérisation selon l'une des revendications 1 à 3, le procédé comprenant deux, trois, quatre ou cinq étages de réaction.

5. Procédé d'oligomérisation selon l'une des revendications 1 à 4, le catalyseur d'oligomérisation utilisé pour l'introduction étant un catalyseur d'oligomérisation nouvellement préparé ou un catalyseur d'oligomérisation régénéré.

6. Procédé d'oligomérisation selon la revendication 5, le catalyseur d'oligomérisation régénéré étant préparé par les étapes A) brûlage d'un catalyseur d'oligomérisation utilisé pour l'oligomérisation et B) imprégnation du catalyseur d'oligomérisation brûlé dans l'étape A) par une solution d'un composé de métal de transition et calcination consécutive.

7. Procédé d'oligomérisation selon l'une des revendications 1 à 6, l'oligomérisation étant réalisée à une température dans la plage de 50 à 200°C et à une pression de 10 à 70 bars.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010057805 A1